(19) Europäisches Patentamt
European Patent Office
Office européen des brevets

(11) Publication number: **0 501 379 A2**

# (12) EUROPEAN PATENT APPLICATION

(21) Application number: **92103067.2**

(51) Int. Cl.[5]: **C07D 473/06**, A61K 31/52

(22) Date of filing: **24.02.92**

(30) Priority: **25.02.91 JP 29796/91**

(43) Date of publication of application:
**02.09.92 Bulletin 92/36**

(84) Designated Contracting States:
**DE FR GB IT**

(71) Applicant: **KYOWA HAKKO KOGYO CO., LTD.**
**6-1, Ohte-Machi Itchome**
**Chiyoda-ku Tokyo(JP)**

(72) Inventor: **Suzuki, Fumio**
**18-4, Fujimidai**
**Mishima-shi, Shizuoka-ken(JP)**
Inventor: **Shimada, Junichi**
**270-1, Fushimi, Shimizu-cho**
**Sunto-gun, Shizuoka-ken(JP)**

(74) Representative: **Vossius & Partner**
**Siebertstrasse 4 P.O. Box 86 07 67**
**W-8000 München 86(DE)**

## (54) Xanthine compounds.

(57) Novel xanthine compounds represented by the following formula:

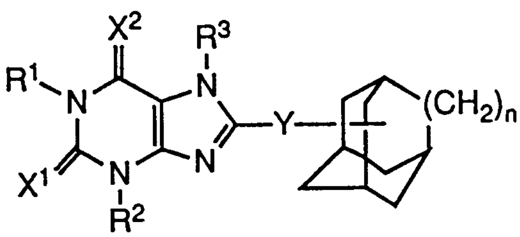

wherein each of $R^1$ and $R^2$ independently represents allyl or propargyl; $R^3$ represents hydrogen or lower alkyl; each of $X^1$ and $X^2$ independently represents oxygen or sulfur; Y represents a single bond or alkylene, and n represents 0 or 1; and pharmaceutically acceptable salts thereof have a diuretic effect and a renal-protecting effect.



Rank Xerox (UK) Business Services

The present invention relates to novel xanthine compounds having a diuretic effect and a renal-protecting effect.

In relation to the compounds of the present invention, 8-(1-adamantyl)-1,3,7-trimethylxanthine is described in Tetrahedron Lett., 27, 6337 (1986). However, nothing is mentioned on its pharmacological effect.

Further, 8-(1-adamantyl)-1,3-dipropylxanthine having an activity of antagonizing adenosine $A_1$ receptor is described in J. Med. Chem., 33, 1906 (1990).

The object of the present invention is to provide novel xanthine compounds exhibiting strong diuretic and renal-protecting effect, based on the finding that xanthine compounds which are adenosine receptor antagonists, particularly those having an activity of selectively antagonizing adenosine $A_1$ receptor, have strong diuretic and renal-protecting effect.

The present invention relates to a xanthine compound represented by the following formula (I):

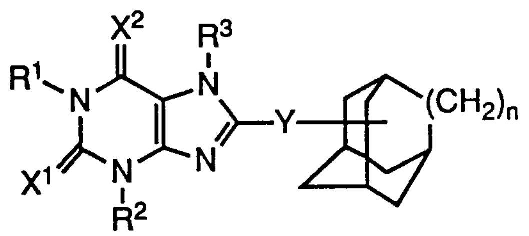

(I)

wherein each of $R^1$ and $R^2$ independently represents allyl or propargyl;
$R^3$ represents hydrogen or lower alkyl;
each of $X^1$ and $X^2$ independently represents oxygen or sulfur; and Y represents a single bond or alkylene, n represents 0 or 1;
[hereinafter referred to as "Compound (I)" and compounds with other formula numbers are likewise referred to], or a pharmaceutically acceptable salt thereof.

In the definition of the respective groups in the formula (I), the lower alkyl includes straight or branched alkyl having 1 to 6 carbon atoms, for example, methyl, ethyl, propyl, isopropyl, butyl, isobutyl, sec-butyl, tert-butyl, pentyl, neopentyl and hexyl. The alkylene includes straight or branched alkylene having 1 to 4 carbon atoms, for example, methylene, ethylene, trimethylene, tetramethylene, methylmethylene, propylene and ethylethylene.

The pharmaceutically acceptable salt of Compound (I) includes pharmaceutically acceptable acid addition salts, metal salts, ammonium salts, organic amine addition salts and amino acid addition salts.

The pharmaceutically acceptable acid addition salt of Compound (I) includes inorganic acid salts,such as hydrochlorides, sulfates and phosphates, and organic acid salts,such as acetates, maleates, fumarates, oxalates and citrates.

The pharmaceutically acceptable metal salt includes alkali metal salts,such as sodium salts, and potassium salts, alkaline earth metal salts,such as magnesium salts, and calcium salts, and also aluminum and zinc salts.

The pharmaceutically acceptable ammonium salt includes salts of ammonium and tetramethylammonium. The pharmaceutically acceptable organic amine addition salt includes addition salts of morpholine and piperidine, and the pharmaceutically acceptable amino acid addition salt includes addition salts of lysine, glycine and phenylalanine.

A process for producing Compound (I) of the present invention is described below.

Compound (I-1) which is Compound (I) wherein $R^3$ is hydrogen, is produced by the following production steps:

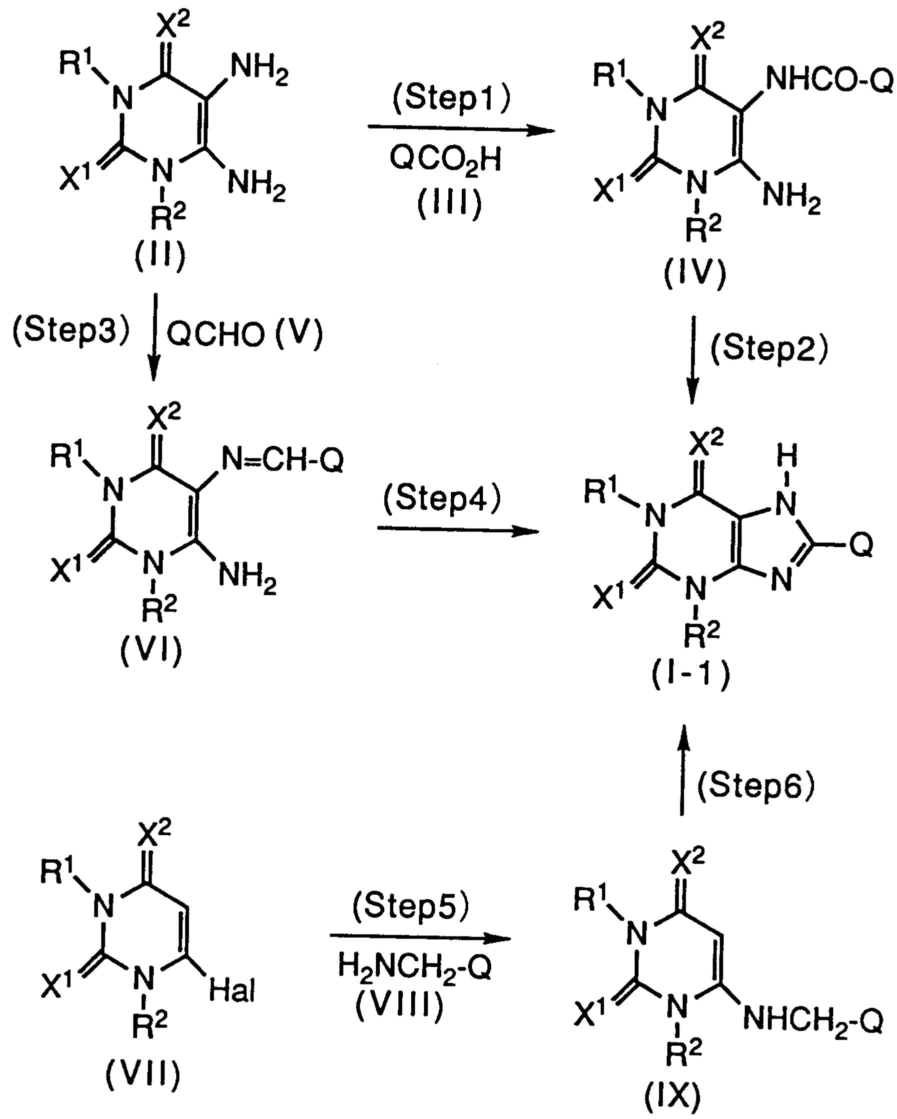


wherein Q represents

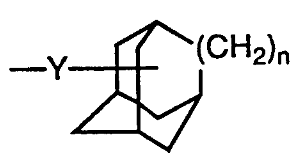

where Y and n have the same meanings as defined above,
Hal represents halogen such as chlorine, bromine or iodine and $R^1$, $R^2$, $X^1$ and $X^2$ have the same meanings as defined above.

Step 1:

A Compound (IV) can be obtained by reacting a uracil derivative (II) obtained according to a well known process, for example, the process disclosed in JP-A- 42383/84, with a carboxylic acid (III) or a carboxylic acid reactive derivative.

The carboxylic acid reactive derivative includes acid halides such as acid chlorides and acid bromides, active esters such as p-nitrophenyl ester and N-oxysuccinimide ester, acid anhydrides commercially available or those formed from carbodiimides such as 1-ethyl-3-(3-dimethylaminopropyl)carbodiimide,

diisopropylcarbodiimide and dicyclohexylcarbodiimide; and mixed acid anhydrides with monoethyl carbonate and monoisobutyl carbonate.

The reaction of Compound (II) with Compound (III) is carried out without any solvent at a temperature of 50 to 200°C. In the case of using the carboxylic acid reactive derivative, the reaction can be carried out according to a process usually used in the peptide chemistry. For example, the reaction solvent is properly selected from halogenohydrocarbons such as methylene chloride, chloroform and dichloroethane, ethers such as dioxane and tetrahydrofuran, dimethylformamide and dimethylsulfoxide, and if necessary, water is used. The reaction temperature is -80 to 50°C, and the reaction is completed for 0.5 to 24 hours. Sometimes, the reaction may be favorably carried out, if necessary, in the presence of an additive such as 1-hydroxybenzotriazole, or a base such as pyridine, triethylamine, dimethylaminopyridine and N-methylmorpholine. Furthermore, the carboxylic acid reactive derivative may be formed in the reaction system and used without isolation.

Step 2:

A desired Compound (I-1) is obtained from Compound (IV) by the reaction in the presence of a base (process A), by treatment with a dehydrating agent (process B), or by heating (process C).

As the preferable base in the process A, alkali metal hydroxides such as sodium hydroxide and potassium hydroxide can be exemplified. As the reaction solvent, water, lower alcohols such as methanol and ethanol, ethers such as dioxane and tetrahydrofuran, dimethylformamide and dimethylsulfoxide can be used alone or in combination. The reaction is carried out at a temperature of from room temperature to 180°C and is usually completed for 10 minutes to 6 hours.

As the dehydrating agent for use in the process B, thionyl halides such as thionyl chloride, and phosphorus oxyhalides such as phosphorus oxychloride can be used, and the reaction is carried out at a temperature of from room temperature to 180°C without any solvent or in a solvent inert to the reaction, for example, halogenohydrocarbons such as methylene chloride, chloroform and dichloroethane, dimethylformamide and dimethylsulfoxide and is usually completed for 0.5 to 12 hours.

In the case of process C, Compound (I-1) can be obtained by heating Compound (IV) at a temperature of 50 to 200°C in a polar solvent such as dimethylsulfoxide, dimethylformamide and Dowthermo A (product of Muromachi Kagaku Kogyo Kaisha, Ltd.).

Step 3:

A Schiff base (VI) can be obtained by reacting Compound (II) with aldehyde (V) in a mixed solvent such as a mixture of acetic acid with a lower alcohol such as methanol and ethanol at a temperature of -20 to 100°C.

Step 4:

A desired Compound (I-1) can be obtained by subjecting Compound (VI) to an oxidative cyclization reaction.

As the appropriate oxidizing agent, oxygen, ferric chloride, ceric (IV) ammonium nitrate and diethyl azodicarboxylate can be exemplified. The reaction is carried out by heating Compound (VI) at a temperature of from room temperature to 180°C in the presence of the afore-mentioned oxidizing agent and, if necessary, in a solvent inert to the reaction, for example, a lower alcohol such as methanol and ethanol, a halogenohydrocarbon such as methylene chloride and chloroform, or an aromatic hydrocarbon such as toluene, xylene and nitrobenzene.

Step 5:

A Compound (IX) can be obtained by reacting a uracil derivative (VII) obtained according to a well known process, for example, the process described in JP-A- 5082/86 with an amine (VIII) in a solvent inert to the reaction, for example, a lower alcohol such as methanol, ethanol, etc., dimethylformamide, dimethylsulfoxide, etc. alone or in combination thereof at a temperature of 50 to 150°C.

Step 6:

A Compound (I-1) can be obtained by reacting a Compound (IX) with a nitrosation agent such as

sodium nitrite and isoamyl nitrite, etc. under an acidic condition with dilute hydrochloric acid in a solvent inert to the reaction, for example, a lower alcohol such as methanol and ethanol usually at a temperature of from room temperature to the boiling point of the solvent.

Step 7:

A Compound (I-2) which is Compound (I) wherein $R^3$ is lower alkyl can be obtained through the following step:

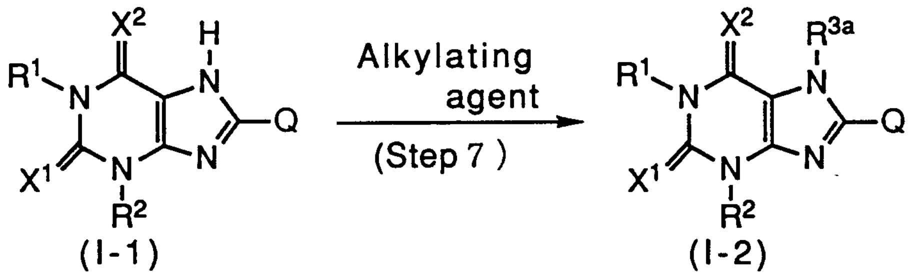


wherein $R^1$, $R^2$, $X^1$, $X^2$ and Q have the same meanings as defined above, $R^{3a}$ represents lower alkyl in the definition of $R^3$.

A desired Compound (I-2) can be obtained by reacting Compound (I-1) obtained in Steps 1 to 6 with an alkylating agent preferably in the presence of a base.

As the alkylating agent, e.g. alkyl halides, dialkyl sulfates, diazoalkanes are used.

As the base, an alkali metal carbonate such as sodium carbonate and potassium carbonate, an alkali metal hydride such as sodium hydride, and an alkali metal alkoxide such as sodium methoxide and sodium ethoxide are exemplified. The reaction is completed at a temperature of 0 to 180 ° C for 0.5 to 24 hours.

Step 8:

Compound (I-4) which is Compound (I) wherein $X^2$ is sulfur, can be obtained by the following step.

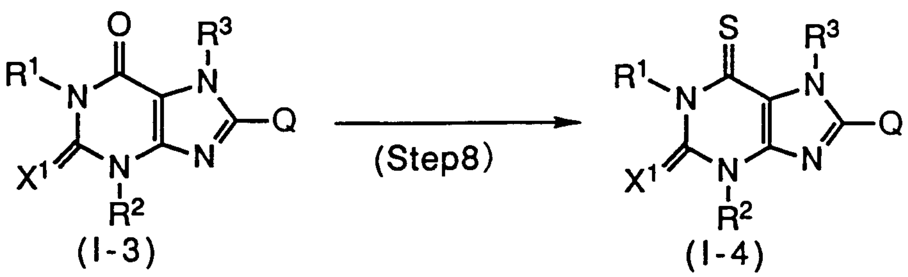


wherein $R^1$, $R^2$, $R^{3}$' $X^1$ and Q have the same meanings as previously defined.

A desired Compound (I-4) was prepared by reacting Compound (I-3) which is Compound (I) wherein $X^2$ is oxygen, with an appropriate thionation reagent, in an inert solvent. As the thionation reagent and phosphorus pentasulfide are mentioned. As the solvent, dimethylformamide, tetrahydrofuran and dioxane are mentioned, and preferably pyridine is used. The reaction is carried out at a temperature of 50 to 180 ° C for a period of 10 minutes to 36 hours.

Step 9:

Compound (I-5) which is Compound (I) wherein $R^3$ is hydrogen and $X^2$ is oxygen, can be obtained by the following step.

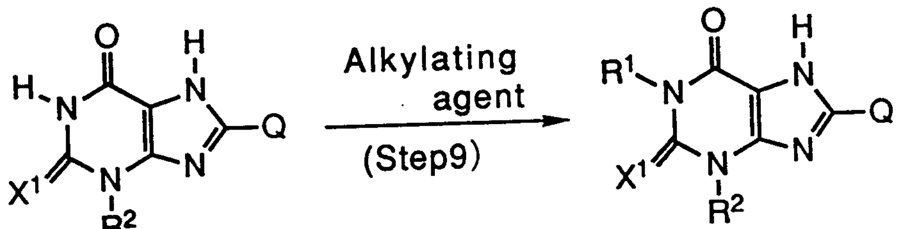


(X)

(I-5)

wherein $R^1$, $R^2$, $X^1$ and Q have the same meanings as previously defined.

Compound (I-5) can be obtained by reacting Compound (X) obtained according to a similar procedure to Steps 1 to 6 with an equimolar amount of an alkylating agent, if necessary, in the presence of a base.

As the alkylating agent, e.g. alkyl halides such as alkyl bromide and propargyl bromide, and sulfonic acid esters such as propargyl p-toluenesulfonate and allyl methanesulfonate are used.

As the base, an alkali metal carbonate such as sodium carbonate and potassium carbonate, an alkali metal hydride such as sodium hydride, and an alkali metal alkoxide such as sodium methoxide and sodium ethoxide are exemplified. As the reaction solvent, lower alcohols such as methanol and ethanol, ethers such as dioxane and tetrahydrofuran, and dimethylformamide and dimethylsulfoxide can be used alone or in combination. The reaction is carried out at a temperature of from room temperature to 180°C and usually completed in from 10 minutes to 6 hours.

The intermediates and desired compounds obtained according to the aforementioned processes can be isolated and purified by subjecting the intermediates and desired compounds to a purification process usually used in the organic synthetic chemistry, for example, filtration, extraction, washing, drying, concentration, recrystallization and various chromatographies. The intermediates can be used in the successive reaction without any purification.

Salts of Compound (I) can be obtained by direct purification when Compound (I) can be obtained in a salt form, or by solving or suspending the free form in an appropriate solution, adding an acid or base to the solution or suspension, and subjecting the mixture to a subsequent purification procedure, when Compound (I) is obtained in a free form.

Compound (I) and its pharmaceutically acceptable salts sometimes exist in an adduct form with water or various other solvents, and these adducts are included in the present invention.

Optical isomers may exist with respect to Compound (I), and all the possible stereoisomers and their mixtures are also included in the scope of the present invention.

Compound (I) and its pharmaceutically acceptable salts have an activity of selectively antagonizing adenosine $A_1$ receptor, and thus exhibit, a diuretic effect and a renal-protecting effect. Compound (I) and its pharmaceutically acceptable salts are useful as a diuretic and renal-protecting agent.

The pharmacological effects of Compound (I) are illustrated, referring to Test Examples.

Test Example 1, acute toxicity test

A test compound was orally administered to male dd-strain mice having a body weight of 20 ± 1 g (3 animals/group). Minimum lethal dose (MLD) of the compound was determined by observing whether or not the mice were alive after 7 days of the administration.

With respect to Compound 1, the MLD was more than 300 mg/kg. This shows the toxicity of Compound (I) is weak and can be administered safely over a wide range of dosage.

Test Example 2, adenosine $A_1$ receptor binding test

This test was conducted according to the method of Bruns et al. [Proc. Natl. Acad. Sci., 77, 5547 (1980)] with some modification.

Cerebrum of a guinea pig was suspended into ice cooled 50 mM tris hydroxymethyl aminomethane hydrochloride (Tris HCl) buffer (pH 7.7), by using Polytron homogenizer (manufactured by Kinematica Co.). The suspension was centrifuged (50,000 x g, 10 minutes), and the precipitate was resuspended by adding the same volume of 50 mM Tris HCl buffer. The suspension was centrifuged under the same conditions, and the precipitate obtained was suspended once again by adding 50 mM Tris HCl until the concentration of tissue was 100 mg (wet weight)/ml. The tissue suspension was incubated at 37°C for 30 minutes in the

presence of 0.02 units/mg tissue of adenosine deaminase (manufactured by Sigma Co.). The resulting tissue suspension was recentrifuged (50,000 x g, 10 minutes), and 50 mM Tris HCl was added to the precipitate until the concentration of tissue was 10 mg (wet weight)/ml.

To 1 ml of tissue suspension prepared above were added 50 $\mu$l of [$^3$H] cyclohexyladenosine [$^3$H-CHA, 27 Ci/mmol, manufactured by New England Nuclear Co.] (final concentration: 1.1 nM) and 50 $\mu$l of the test compound. The mixture was incubated at 25 °C for 90 minutes, and the resulting mixture was stopped by rapid vacuum filtration through a glass fiber filter (GF/C manufactured by Whatman Co.) and immediately washed three times with 5 ml each of ice cold 50 mM Tris HCl buffer. The filter was transferred to a vial bottle, and a scintillator (EX-H by Wako Pure Chemicals Industries, Ltd.) was added thereto. Its radioactivity was then determined by a scintillation counter (manufactured by Packard Instrument Co.).

The inhibition rate of the test compound against the binding of $A_1$ receptor ($^3$H-CHA binding) was calculated from the following equation:

$$\text{Inhibition (\%)} = \left(1 - \frac{[B] - [N]}{[T] - [N]}\right) \times 100$$

[Notes]

1. "B" means the radioactivity of $^3$H-CHA bound in the presence of a test compound at a concentration shown in Table 1.
2. "T" means the radioactivity of $^3$H-CHA bound in the absence of a test compound.
3. "N" means the radioactivity of $^3$H-CHA bound in the presence of 10 $\mu$M of $N^6$-(L-2-phenylisopropyl)-adenosine (manufactured by Sigma Co.).

The results are shown in Table 1. The inhibition constant (Ki value) shown in the table was calculated from Cheng-Prusoff's equation.

Table 1

| Compound No. | $A_1$ Receptor | |
|---|---|---|
| | Inhibition (%)/ Concentration of Test Compound [$10^{-5}/10^{-4}$ M] | Ki (nM) |
| 1 | 99/99 | 15 |

According to the result, Compound 1 almost perfectly inhibited against $^3$H-CHA binding (the binding of $A_1$ receptor).

Test Example 3, diuretic effect

Wistar rats (male: 150-300 g) were starved for 18 hours prior to the administration of a test compound. The test compound was suspended in saline, and the test compound was orally administered to rats at a concentration of 0.1 to 10 mg/25 ml/kg. Only saline was administered to rats. The rats to which only saline was administered was made as control group. Three groups, each group consisting of 3 rats, were used for each test Urine was collected for 6 hours after the administration. Urine volume was measured and the electrolytes ($Na^+$ and $K^+$) in the urine were determined with a flame photometer (775A, Hitachi Ltd., Japan). The results are shown in Table 2.

All parameters are expressed as relative values of control.

Table 2

| Compound No. | The amount of the administration (mg/kg) | Increase in Urine (%) | Increase in $Na^+$ excretion (%) | Increase in $K^+$ excretion (%) | $Na^+/K^+$ |
|---|---|---|---|---|---|
| (Control) | -- | 0 | 0 | 0 | 1.00 |
| 1 | 6.25 | 82 | 71 | 18 | 1.45 |
| 1 | 0.40 | 109 | 99 | 8 | 1.84 |
| Aminophylline*1 (Reference compound) | 25 | 34 | 89 | 17 | 1.62 |
| Furosemide*2 (Reference compound) | 25 | 75 | 64 | 57 | 1.07 |

*1 The Merck Index, 11th edition, page 76 (1989)

*2 The Merck Index, 11th edition, page 674 (1989)

The result indicates that diuretic effect of Compound 1 is higher than that of Aminophylline or Furosemide.

Test Example 4,renal-protecting effect (glycerol-induced renal failure model)

A renal failure is a state where the renal function is lowered and the homeostasis of a body fluid can be no more maintained. It is known that an acute renal failure characteristic of uriniferous tubule disorder is caused by subcutaneous or intramuscular injection of glycerol to rats [Can. J. Physiol. Pharmacol., 65, 42 (1987)].

Male Wistar rats were kept deprived of water for 18 hours, and served for the test. A test compound was intraperitoneally administered to the rats (dosage: 0.1 mg/1 ml/(saline)/kg) and the rats were anesthetized with ether and 50% glycerol was subcutaneously administered (dosage: 0.8 mg/100 g) to the rats, pinching the dorsal skin. Twenty four hours after the administration of glycerol, the rats were anesthetized with ether and 5 ml of blood was collected from the abdominal aorta. The collected blood was allowed to stand for 30 minutes or longer and then centrifuged at 3,000 rpm for 10 minutes, and the amounts of the serum creatinine and the serum urine-nitrogen (UN) were determined by auto analyzer (Olympus AU510) [creatinine test (Jaffé method), UN test (enzyme method); both tests were used in Olympus AU500/550 exclusive reagent] or measured by the Creatinine Test Wako (Jaffé method) and UN Test Wako (diacetylmonooxime direct method). Both are manufactured by Wako Pure Chemicals Co.

On the other hand, the left kidneys of the blood-sampled rats were removed and placed in formalin-filled vial bottles, and pathologically examined in contrast with the left kidneys of test compound-untreated rats.

The results are shown in Table 3.

Table 3

| Compound No. | The amount of the administration (mg/kg) | The amount of the serum creatinine (mg/dl) | | The amount of the serum urine-nitrogen (mg/dl) | |
|---|---|---|---|---|---|
| | | Control group | Test compound administered-group | Control group | Test compound administered-group |
| 1 | 0.1 | 4.94±0.05 | 2.15±0.22(***) | 174.8±4.0 | 78.6±9.7(***) |
| Aminophylline (Reference compound) | 10 | 2.03±0.18 | 1.72±0.07 | 46.2±6.5 | 30.6±2.0(**) |
| Furosemide (Reference compound) | 10 | 3.22±0.35 | 4.17±0.41 | 110.7±9.4 | 150.3±13.7(**) |
| Control | -- | 0.50±0.02 | -- | 15.2±0.9 | -- |

(***: $P < 0.001$, **: $P < 0.05$)

Values in test compound-treated group were compared to those in control group using the Student's t-test (n = 8 ~ 10).

According to the results, Compound 1 significantly suppressed increases in the serum creatinine and in serum urine-nitrogen, when administered abdominally at a dosage of 0.1 mg/kg [i.p.] whereas aminophylline (10 mg/kg) had a weak effect of suppressing the increase. On the contrary, furosemide (10 mg/kg) showed a tendency to increase the serum creatinine. The pathological examination of removed kidney indicates that Compound 1 also significantly improved the state of kidney.

Compound (I) or its pharmaceutically acceptable salts can be used as such or in various medicament forms. The present pharmaceutical composition can be prepared by uniformly mixing an effective amount of Compound (I) or its pharmaceutically acceptable salts as an active ingredient with a pharmaceutically acceptable carrier. The pharmaceutical composition is desirably in a unit dosage form applicable to oral or injection administration.

In the preparation of pharmaceutical compositions in an oral dosage form, some appropriate, pharmaceutically acceptable carrier can be used. For example, liquid, orally administerable compositions such as suspension compositions and syrup compositions can be prepared with water, a saccharide such as sucrose, sorbitol and fructose, a glycol such as polyethyleneglycol and propyleneglycol, an oil such as sesame oil, olive oil and soybean oil, an antiseptic such as p-hydroxybenzoic acid esters, and a flavor such as strawberry flavor and peppermint. Powder, pills, capsules and tablets can be prepared with a vehicle such as lactose, glucose, sucrose and mannitol, a disintegrator such as starch and sodium alginate, a lubricant such as magnesium stearate and talc, a binder such as polyvinyl alcohol, hydroxypropyl cellulose and gelatin, a surfactant such as fatty acid esters, a plasticizer such as glycerin. Tablets and capsules are most useful unit for oral administration because of easy administration. In the preparation of tablets or capsules, a solid pharmaceutical carrier is used.

Solutions, suspensions or dispersing solutions for injection can be prepared with a carrier such as distilled water, saline solution, glucose solution, or a mixture of saline solution and glucose solution.

Effective dosage and number of administration of Compound (I) or its pharmaceutically acceptable salts depend on the administration route and ages, body weights and symptoms of patients, and it is preferable to usually administer Compound (I) at a dosage of 1 to 50 mg/kg per day in 3 to 4 divisions.

The present invention is described below, by the following Examples and Reference Examples.

Example 1

1,3-Diallyl-8-(3-noradamantyl)xanthine (Compound 1)

At first, 1.65 g (9.91 mmols) of 3-noradamantanecarboxylic acid was dissolved in 20 ml of pyridine, and 0.80 ml (10.8 mmols) of thionyl chloride was dropwise added thereto at 0° C. The mixture was stirred for 1 hour at room temperature, and cooled to 0° C once again. 2.00 g (9.01 mmols) of 5,6-diamino-1,3-diallyluracil [Naunyn-Schmiedeberg's Arch. Pharmacol., 336, 204 (1987)] in 20 ml of a methylene chloride solution was dropwise added. The reaction mixture was further stirred for 1 hour at room temperature, and concentrated under reduced pressure. The concentrate was poured into 100 ml of water. The mixture was extracted with 30 ml of chloroform three times. The organic layers were combined, washed with a saturated aqueous sodium chloride, and dried over anhydrous sodium sulfate. The solvent was evaporated under reduced pressure. The residue was purified and isolated by silica gel column chromatography (eluent: 2% methanol/chloroform) to afford 2.44 g (yield: 73%) of 6-amino-1,3-diallyl-5-(noradamantane-3-carbonylamino)uracil as amorphous.

NMR (90 MHz; $CDCl_3$) δ (ppm):

7.41 (1H, brs), 6.20~5.10 (8H, m), 4.80~4.45 (4H, m), 2.76 (1H, t), 2.50~1.50 (12H, m)

Then, 27 ml of 2N sodium hydroxide aqueous solution and 14 ml of dioxane were added to 2.29 g (6.19 mmols) of the obtained compound and the mixture was refluxed under heating for 1 hour. After cooling, the reaction mixture was neutralized with concentrated hydrochloric acid, and extracted with chloroform three times. Then the organic layers were combined, washed with a saturated aqueous sodium chloride and dried over anhydrous sodium sulfate. The solvent was evaporated under reduced pressure. The obtained crude crystals were recrystallized from isopropanol-water to afford 1.55 g (yield: 71%) of Compound 1 as a white needle crystal.

Melting point:

202.0-204.3° C

| Elemental analysis: $C_{20}H_{24}N_4O_2$ | | | |
|---|---|---|---|
| Calculated (%) | C 68.16, | H 6.86, | N 15.89 |
| Found (%) | C 68.20, | H 6.97, | N 15.63 |

IR (KBr) $\nu_{max}$ ($cm^{-1}$):

3190, 2940, 1704, 1658, 1651, 1550, 1498

NMR (90 MHz; $CDCl_3$) δ (ppm):

6.30~5.70 (2H, m), 5.60~5.00 (4H, m), 4.90~4.60 (4H, m), 2.80 (1H, t), 2.55~1.50 (12H, m)

MS (m/e): 352 ($M^+$)

Example 2

3-Allyl-8-(3-noradamantyl)-1-propargylxanthine (Compound 2)

At first, 1.10 g (3.21 mmols) of 3-allyl-8-(3-noradamantyl)xanthine obtained in Reference Example 1 was dissolved in 32 ml of dimethylformamide, and 257 mg (6.42 mmols) of 60% sodium hydride was added at 0° C. After the mixture was stirred for 30 minutes at room temperature, 0.25 ml (3.36 mmols) of propargyl bromide was dropwise added thereto. The mixture was stirred for 4 hours at room temperature, and the reaction mixture was poured into 300 ml of water and was neutralized with 1N hydrochloric acid. The mixture was extracted with 50 ml of chloroform three times. The organic layers were combined, washed with water twice and then with a saturated aqueous sodium chloride, and dried over anhydrous sodium sulfate. The solvent was evaporated under reduced pressure. The residue was purified and isolated by flush chromatography (eluent: 40% ethyl acetate/hexane) and recrystallized from dimethylsulfoxide-water to afford 110 mg (yield: 23%) of Compound 2 as a white powder.

Melting point:

256.3~257.1° C

| Elemental analysis: $C_{20}H_{22}N_4O_2$ | | | |
|---|---|---|---|
| Calculated (%) | C 68.55, | H 6.32, | N 15.98 |
| Found (%) | C 68.62, | H 6.45, | N 16.05 |

IR (KBr) $\nu_{max}$ ($cm^{-1}$):
1700, 1659, 1649, 1548, 1496
NMR (270 MHz; $CDCl_3$) $\delta$ (ppm):
11.58 (1H, brs), 6.10~5.90 (1H, m), 5.45~5.20 (2H, m), 4.90~4.70 (4H, m), 2.80 (1H, t), 2.50~1.90 (8H, m), 1.80~1.60 (5H, m).
MS (m/e): 350 ($M^+$)

Reference Example 1

3-Allyl-8-(3-noradamantyl)xanthine

At first, 3.22 g (19.4 mmols) of 3-noradamantanecarboxylic acid was dissolved in 80 ml of pyridine, and 1.54 ml (21.1 mmols) of thionyl chloride was dropwise added under ice-cooling. The mixture was stirred for 50 minutes at room temperature. 3.21 g (17.6 mmols) of 1-allyl-5,6-diaminouracil (U.S. Patent No. 2,673,848) was gradually added to the reaction mixture under ice-cooling. After stirring for 2 hours at room temperature, the reaction mixture was concentrated under reduced pressure. The residue was extracted with chloroform/methanol (5/1) five times. The organic layers were combined and dried over anhydrous sodium sulfate. The solvent was evaporated under reduced pressure. 30 ml of dioxane and 60 ml of aqueous 1N sodium hydroxide solution were added to the residue, and the mixture was refluxed under heating for 30 minutes. After cooling, the reaction mixture was neutralized with 1N hydrochloric acid, and was extracted with 50 ml of chloroform three times. The organic layers were combined, washed with a saturated aqueous sodium chloride and dried over anhydrous sodium sulfate. The solvent was evaporated under reduced pressure to afford 4.92 g (yield: 90%) of the captioned Compound as a light yellow plate crystal.
Melting point:
> 270 °C (recrystallized from ethanol/water)

| Elemental analysis: $C_{17}H_{20}N_4O_2$ | | | |
|---|---|---|---|
| Calculated (%) | C 65.36, | H 6.45, | N 17.93 |
| Found (%) | C 64.98, | H 6.72, | N 17.86 |

IR (KBr) $\nu_{max}$ ($cm^{-1}$):
1685, 1648, 1643, 1498, 1425
NMR (90 MHz; $CDCl_3$) $\delta$ (ppm):
12.10 (1H, brs), 7.20 (1H, s), 6.20~5.65 (1H, m), 5.45~5.05 (2H, m), 4.80~4.45 (2H, m), 2.71 (1H, t), 2.55~1.50 (12H, m)

Pharmaceutical preparation 1

Tablet:

A tablet having the following composition was prepared according to the conventional method.

| | |
|---|---|
| Compound 1 | 20 mg |
| Lactose | 60 mg |
| Potato starch | 30 mg |
| Polyvinyl alcohol | 3 mg |
| Magnesium stearate | 1 mg |

## Claims

1. A xanthine compound represented by the following formula (I):

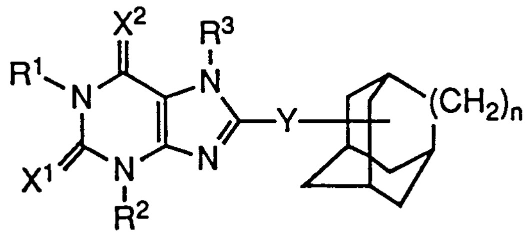

(I)

wherein each of $R^1$ and $R^2$ independently represents allyl or propargyl;
$R^3$ represents hydrogen or lower alkyl;
each of $X^1$ and $X^2$ independently represents oxygen or sulfur;
Y represents a single bond or alkylene, and
n represents 0 or 1;
or a pharmaceutically acceptable salt thereof.

2. The xanthine compound according to Claim 1, wherein $X^1$ and $X^2$ are both oxygen; and n is 0.

3. 1,3-Diallyl-8-(3-noradamantyl)xanthine, and a pharmaceutically acceptable salt thereof.

4. 3-Allyl-8-(3-noradamantyl)-1-propargylxanthine and a pharmaceutically acceptable salt thereof.

5. A pharmaceutical composition comprising a pharmaceutical carrier and, as an active ingredient, an effective amount of a xanthine compound as defined by Claim 1 or a pharmaceutically acceptable salt thereof.